# EUROPEAN PATENT APPLICATION

(11) **EP 1 992 385 A2**
(43) Date of publication of application: **19.11.2008**
(21) Application number: 08003351.7
(22) Date of filing: 16.10.2002
(51) Int. Cl.: A61M 31/00

(54) **A disposable device for transferring and/or circulating an active liquid into and/or inside an intracorporeal cavity**

(30) Priority: 16.10.2001 US 329293 P; 16.10.2001 FR 0113343; 16.10.2001 US 329332 P; 08.11.2001 US 331127 P; 23.01.2002 US 350041 P; 29.08.2002 US 406638 P
(62) Divisional of application: 02785697.0
(71) Applicant: Chaffringeon, Bernard, 1306 Daillens (CH)
(72) Inventor: Chaffringeon, Bernard, 1306 Daillens (CH)
(74) Representative: Chevalier, Renaud Philippe

(57) **Abstract**

A disposable device for transferring and/or circulating an active liquid Into and/or inside an Intracorporeal cavity Is elongate along a reference axis, has an intrinsic axial consistency which is sufficient to allow the device to be introduced into the cavity by being pushed in axially. The device includes an expandable tampon made of at least one absorbent material, having a proximal portion and a distal portion, designed to collect and absorb said active liquid dispensed in the intracorporeal cavity and to expand and bear In a leak tight manner against mucous membrane of the intracorporeal cavity, and a source of liquid phase including a capsule, at least the proximal part of which is at least partially surrounded by the expandable tampon.

## Description

This nonprovisional application claims the benefit of U.S. Provisional Applications No. 60/329,293, filed October 16, 2001, No. 60/329,332, filed October 16, 2001, No. 60/331,127, filed November 8, 2001, No. 60/350,041, filed January 23, 2001, and No. 60/406,638, filed August 29, 2002, the entire contents of each of which is hereby incorporated by reference.

### FIELD OF THE INVENTION

The present invention concerns the transfer and/or circulation of an active liquid into/inside an intracorporeal cavity of the human body or of an animal, especially in contact with mucous membrane of said cavity.

### BACKGROUND AND DEFINITIONS

"Intracorporeal cavity" signifies any cavity of the body, particularly an elongate one, which can be accessed from the outside for different purposes, in particular for clinical, therapeutic, prophylactic or diagnostic purposes, but also for cosmetic or personal hygiene purposes. One example of such a cavity is the vagina of a woman, which extends from the vulva to the neck of the uterus or cervix, and into/inside which it is desired to transfer and/or circulate an active liquid in contact with the mucous membrane and/or the tissues of said cavity.

"Active liquid" signifies any liquid or fluid for local treatment or delivery, by a topical route, and/or for systemic treatment or delivery, comprising in a general manner a liquid or fluid phase in which an active agent is dispersed, for example in solution or suspension.

This active agent may be at least one compound or substance selected from the group consisting of wetting agents, solubilizing and liquefying agents, in particular of a bodily liquid or fluid present in the intracorporeal cavity, therapeutic agents against infectious and non infectious diseases, prophylactic agents, diagnostic agents, immunotherapeutic agents, cosmetic or personal hygiene agents, antiseptic agents, bactericidal agents, fungicidal agents, and spermicidal agents (in the case of the vaginal cavity of the woman, for example), local treatment agents, systemic treatments agents, trophic agents and lubricant agents.

The liquid or fluid phase (depending on its viscosity) of said active liquid may comprise at least any liquid or natural fluid (such as a body fluid) originally present or naturally generated or secreted in said intracorporeal cavity, if any.

In accordance with the embodiment shown in Figure 4 of International Patent Application Publication No. WO 00/45887, a disposable device has been described and proposed for transferring and/or circulating an active liquid into/inside an intracorporeal cavity, said device being elongate along a reference axis and having an intrinsic axial or longitudinal consistency which is soft but stiff or strong enough to allow it to be introduced into said cavity by being pushed in axially, without altering the structure of the device, particularly without dissociating its various components and/or rupturing a capsule, if any.

This device comprises:
- a distal capsule, generally in the shape of a pear, having a relatively thick wall made of a liquefiable substrate which is relatively solid at ambient temperature, that is to say outside the intracorporeal cavity, and which becomes relatively liquid inside said intracorporeal cavity; in this way, the substrate at least partially liquefies inside said intracorporeal cavity and the wall of said capsule disrupts; this capsule comprises or incorporates a liquid charge, enclosed by the aforementioned wall in a leak tight manner relative to the outside of the capsule; an active agent as defined above is distributed in the substrate and/or the liquid charge;
- a proximal expandable tampon, generally with the shape of an egg, made of an absorbent material, joined to the aforementioned capsule and designed to collect and absorb said active liquid dispensed in the intracorporeal cavity and, in time, to expand and bear in a leak-tight manner against the mucous membrane of the intracorporeal cavity; this tampon is obtained from a porous material such as an open-pore foam of a suitable synthetic resin, or alternatively cellulose wadding;
- a covering for temporary protection enveloping the entire capsule and the tampon which are joined to each other, covering the proximal end of the tampon and the distal end of the capsule.

Thus, according to this publication and also to the present invention as hereunder defined and described:
- the liquid or fluid phase of said active liquid comprises, in addition to any natural liquid or fluid originally and possibly present in the intracorporeal cavity, at least part of the liquid charge enclosed by the capsule of the device, and possibly at least part of the liquefied form of any liquefiable substrate making part of the device, for instance of which the walls of the capsule are made ;
- any liquefiable substrate means any matter, substance, compound or composition, originally or naturally occurring in solid form, for instance at ambient temperature and/or outside said intracorporeal cavity, and capable of any transformation into a liquid or fluid form by any phenomenon, such as melting and/or dissolution, at least in part, etc., under one or more conditions prevailing in said intracorporeal cavity, such as the body temperature, the presence of a body fluid or liquid, a specific pH condition, etc.; examples of such liquefiable substrates include sucrose, gelatine and alginates.

Where the active agent is hydrophilic, the liquid phase is preferably hydrophilic, or where the active agent is hydrophobic or lipophilic the liquid phase is preferably hydrophobic or lipophilic.

For example, the active liquid may be a solution, a suspension, an emulsion, a colloid or other fluid.

### SUMMARY OF THE INVENTION

One object of this invention is a device as defined above, which can be maintained in position in the intracorporeal cavity, once and as soon as said device is inserted into said cavity.

A further object of this invention is a device allowing the active liquid to efficiently spread over the mucous membrane of the intracorporeal cavity, before being absorbed by the tampon of the same device.

With a specific reference to the vagina, a further object of this invention is a device allowing a specific treatment of the cervix.

A further object of the present invention is to provide a disposable device allowing better availability of the active agent.

Another object of the present invention is to provide a non-traumatic device with ease of use for the end user or patient.

Another object of the present invention is to provide a device which is relatively simple to manufacture and mass-produce.

Individual ones and combinations of two or more and in embodiments all of these objects are generally served by a disposable device for transferring and/or circulating an active liquid into and/or inside an intracorporeal cavity, said device being elongate along a reference axis, said device having an intrinsic axial consistency which is sufficient to allow said device to be introduced into said cavity by being pushed in axially, said device comprising:
- an expandable tampon made of at least one absorbent material, having a proximal portion and a distal portion, designed to collect and absorb said active liquid dispensed in the intracorporeal cavity and to expand and bear in a leak-tight manner against mucous membrane of the intracorporeal cavity;
- a source of liquid phase comprising a capsule at least the proximal part of which is at least partially surrounded by said expandable tampon;
- a covering for temporary protection at least partially covering the distal portion of said capsule, and extending longitudinally to an intermediate level of the tampon in such a way as to form in said tampon a proximal plug not covered by said covering, which is at least radially expandable independently from the remaining part of said tampon.

The present invention has two especially preferred types of embodiments, i.e. :
- a first embodiment, wherein said capsule has a wall made of a liquefiable substrate which is relatively solid at ambient temperature, outside the intracorporeal cavity, and which becomes relatively liquid inside said intracorporeal cavity, so that said substrate at least partially liquefies inside said intracorporeal cavity and said wall disrupts, and wherein said capsule has a liquid charge, enclosed by said wall in a leak-tight manner relative to the outside of said capsule, an active agent being distributed in a least one of said substrate and said liquid charge.
- a second embodiment, wherein said capsule comprises a plastic envelope containing a liquid charge of said liquid phase enclosed by said envelope in a leak-tight manner relative to the outside of said capsule, an active agent being distributed in said liquid charge.

Other particular or specific objects of this invention are met by embodiments of a device as described herein, and comprising at least one of the following limitations, considered independently from one another:
(a) the expandable tampon is designed to expand radially (inward and/or outward), and possibly longitudinally or axially;
(b) the distal portion of the tampon is divided in at least two parts, or tongues, that are independently and possibly in a differentiated manner, axially expandable;
(c) there is provided an absorption barrier that reduces absorption of said active liquid dispensed in the intracorporeal cavity by at least a portion of the tampon; preferably this absorption barrier is a coating of a repelling substance, not miscible with said active liquid, coating at least said portion of the tampon, and said repelling substance is hydrophobic or lipophilic where said active liquid is hydrophilic, or hydrophilic where said active liquid is hydrophobic or lipophilic;
(d) the tampon comprises at least one transverse capillary flow isolating section;
(e) the tampon has an assembled composition made of at least a distal section comprising tongues, a proximal section comprising the proximal plug, and an intermediate section between said distal and proximal sections, enclosed in a ring of a liquefiable substrate.

According to the present invention, there is also described a method of transferring and/or circulating an active liquid into and/or inside an intracorporeal cavity, comprising inserting a device as described herein into and/or in contact with the mucous membrane of the intracorporeal cavity, and allowing a liquid phase to be released in said cavity.

The present invention also concerns an apparatus for making a device as previously defined, comprising:
- means for radially compressing at least a first portion of an expandable tampon,
- means for axially compressing at least a second portion of said expandable tampon,
- means for providing said expandable tampon with a source of liquid.

### BRIEF DESCRIPTION OF THE DRAWINGS

The present invention is now described with reference to the attached drawings in which:
- Figure 1 shows, in diagrammatic form, a cross section through a device according to a first embodiment of the invention;
- Figure 2 shows, in diagrammatic form and in cross section, the device shown in Figure 1, in position in a vaginal cavity;
- Figures 3 to 6 illustrate the different phases of functioning of a device according to the first embodiment of invention, once it has been placed in an intracorporeal cavity;
- Figures 7 and 8 and Figures 9 and 10 shows in part, respectively, a device according to a second embodiment of the invention;
- Figure 11 shows, in diagrammatic form and in cross section, a device as is shown in Figures 7 and 8 or 9 and 10, in position in a vaginal cavity;
- Figure 12 shows, in diagrammatic form and in cross section, a device according to a third embodiment of the present invention;
- Figures 13 and 14 show alternative embodiments of devices similar to those shown in Figures 7-10;
- Figure 15 shows the cross-section of an expandable tampon formed according to the invention;
- Figure 16 shows an exemplary apparatus for forming an expandable tampon of the invention;
- Figure 17 shows an alternative compression device that may be used in forming an expandable tampon of the invention;
- Figure 18, in a manner similar to Figure 9 but in axial cross section, shows the mode of impregnation of a tampon belonging to a device according to the present invention, before its positioning in the intracorporeal cavity;
- Figure 19, in a manner similar to Figure 18, shows the tampon in the state when impregnated by the active liquid;
- Figure 20 shows a variant of the first embodiment according to the present invention;
- Figure 21 is a side perspective view of a tampon showing a string arrangement of the invention;
- Figure 22 is a cross-sectional view of the tampon of Figure 21;
- Figure 23 is a cross-sectional and split view, in diagrammatic form, but without any capsule, of a further embodiment of an invention device;
- Figure 24 represents, similarly to Figure 23, the device shown in the same figure, but in its expanded configuration.
- Figure 25 represents in a schematic and comparative manner two different embodiments of a device according to the invention, in its expanded configuration within a female vagina; on the left part, a device according to Figure 1 is shown, whereas on the right part a device according to Figure 18 is shown.

### DETAILED DESCRIPTION OF EXEMPLARY EMBODIMENTS

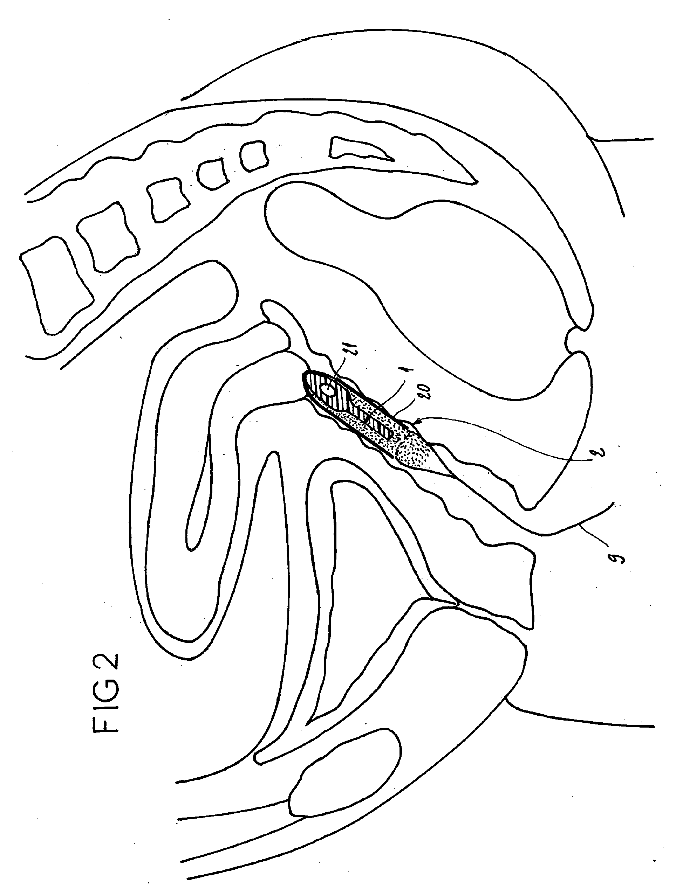

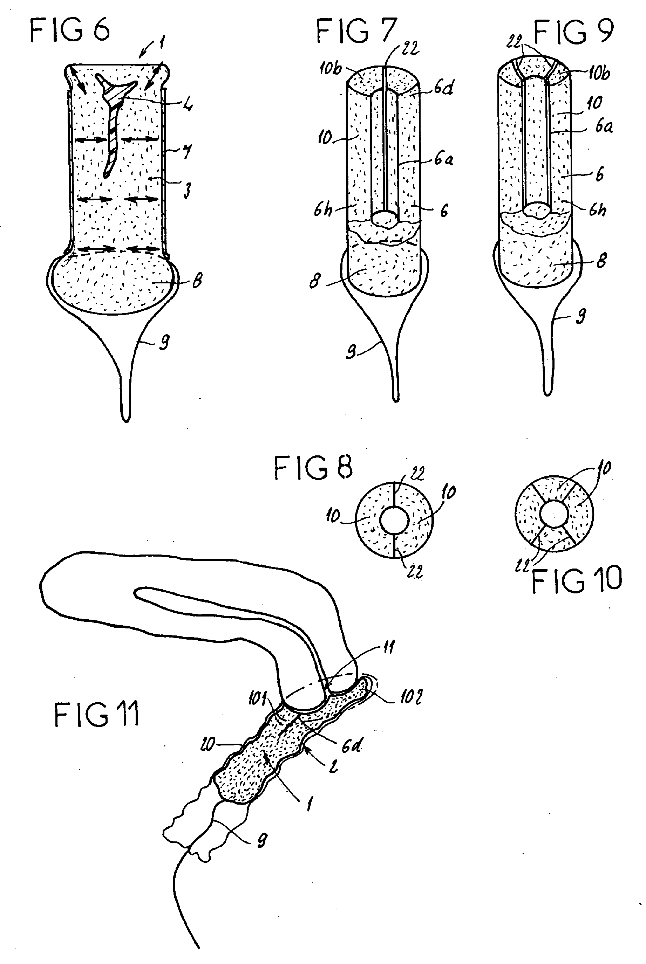

A disposable device 1 according to the invention generally permits the transfer and/or circulation of an active liquid into/inside an intracorporeal cavity 2, for example a vaginal cavity (see Figures 2 and 11).

This device, elongate on a reference axis 15, has an intrinsic axial or longitudinal consistency which is sufficient to allow its introduction into the aforementioned cavity by being pushed in axially.

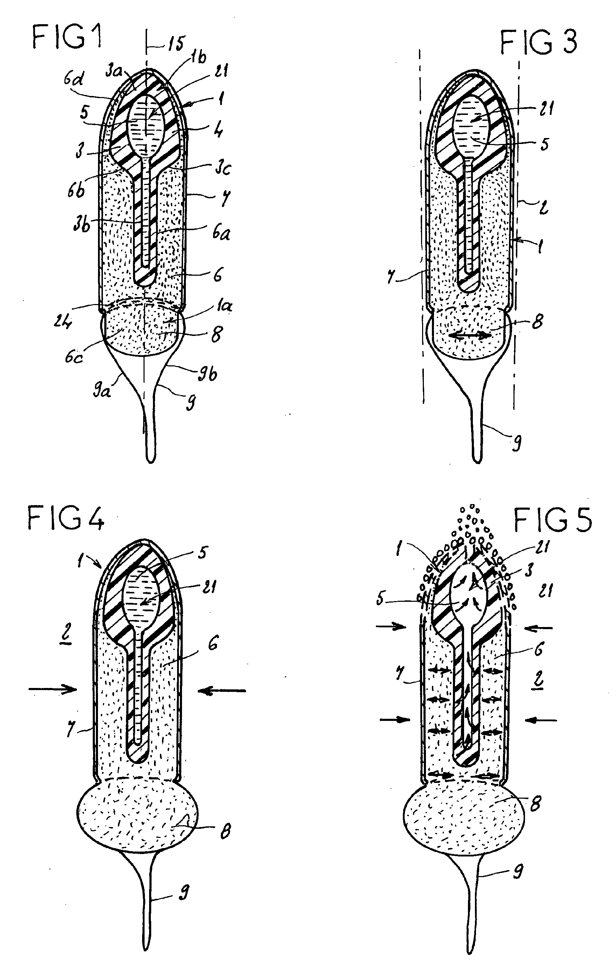

The device of Figure 1 comprises:
- a source 21 of liquid phase comprising a distal capsule 3 having a relatively thick wall 4, the latter being made of a liquefiable substrate which is relatively solid at ambient temperature, that is to say outside the intracorporeal cavity, and which becomes relatively liquid inside this same cavity, that is to say at the temperature of the human or animal body;
- a proximal expandable tampon 6 joined in an appropriate manner to the capsule 3 and designed to collect and absorb active liquid dispensed in the intracorporeal cavity and to expand and bear in a leak-fight manner against the mucous membrane 20 of the intracorporeal cavity 2;
- a covering 7 for temporary protection enveloping the capsule 3 and the tampon 6 which are joined to each other, at least partially covering the distal end of the capsule 3;
- a withdrawal string 9 used for extracting the device 1 and inserted in a loop shape in the tampon 6.

The tampon 6 and the capsule 3 are preferably tailored to one another so that they fit together by simple axial pushing of the capsule toward the tampon, or vice versa.

By virtue of the choice of substrate used for the wall 4 constituting the capsule 3, for example alginate, this at least partially liquefies in the intracorporeal cavity, and the wall 4 disrupts, leaving a passage to the liquid charge 5 originally enclosed by said wall in a leak-tight manner relative to the outside of the capsule 3.

Upon or after liquefying, the substrate may or may not form a further part, in addition to the liquid charge, of the liquid phase for sustaining or making the active liquid transferred and/or circulated into and/or inside the intracorporeal cavity 2.

At least an active agent as defined above is discretely distributed, e.g. dissolved and/or suspended in the liquid charge 5. Alternatively, this active agent can be distributed in the liquefiable substrate of the wall of the capsule 3.

The capsule 3 encloses a liquid charge 5 in a leak-tight manner relative to the outside of the capsule 3, and comprises a wall 4 forming a head 3a and also an axial tail 3b of smaller transverse internal section than the head 3a. Correspondingly, the tampon 6 comprises an axial hole 6a for receiving the tail 3b of the capsule 3. As is shown in Figure 1, the head 3a has a convex proximal part 3c and, correspondingly, the axial hole 6a of the tampon 3 opens out onto a widened distal part 6b for receiving the head 3a.

In the same way, and preferentially, the covering 7 for temporary protection is made of a substrate which is relatively solid at ambient temperature, that is to say outside the intracorporeal cavity 2, and which becomes relatively liquid inside this intracorporeal cavity, so that this covering 7 is able to split open by melting and/or dissolution once the device 1 is placed in the intracorporeal cavity 2.

The covering 7 for temporary protection extends longitudinally or axially to an intermediate but nonetheless proximal level of the tampon 6, in such a way as to form a proximal protective plug 8 not covered by the protective covering 7.

The substrate of the wall 4 of the capsule 3 and/or the substrate of the covering 7 for temporary protection is preferably made of an alginate, or sodium alginate, alternatively called sodium polymannuronate. Such a compound in the form of a solid gel is able to become liquid upon contact with aqueous phase in which it dissolves, e.g. a liquid or fluid, such as a body fluid, which may be present in the intracorporeal cavity 2.

In addition this substrate is biocompatible in the sense that if it contacts the mucous membrane 20 of the intracorporeal cavity 2 it does not induce any significant toxicity and/or inflammatory reaction.

The tampon 6 is designed to expand radially and possibly longitudinally; in the radial dimension, in view of the axial hole 6a, it is designed to expand inward and/or outward. This tampon is made of one or more different materials or parts, at least one of which has an absorbing capacity; such an absorbing material is for example an open-pore foam of a plastic resin, for example polyurethane, or a fibrous material of cellulose such as a cellulose viscose sponge. The tampon 6 can be made by molding, shaping or extruding fibers, filaments, for instance natural fibers.

The expandable tampon 6 is preferably arranged in a compressed manner, e.g., radially and/or axially compressed at least in areas of the tampon, inside the temporary protection covering 7.

As represented by Figure 1, the expandable tampon 6 is a sleeve molded or shaped from one absorbent material, for instance a cellulose viscose sponge. The tampon has a proximal portion 6c and a distal portion 6d that may or may not extend as far distally as shown in Figure 1. For example, it may extend only to the convex proximal part 3c of the head 3a of the capsule 3. Its overall absorbing capacity is designed, in terms of volume and/or predetermined and prior compression, so as to collect and absorb at least part and optionally the totality of the active liquid dispensed in the intracorporeal cavity following the action, or working of the device 1. The tampon 6 surrounds at least the proximal part, comprising the axial tail 3b of the capsule 3, leaving the distal part, portion or end of the same capsule directly in contact with the covering 7.

As described above, the protective covering 7 shown in Figure 1 frees a proximal part of the tampon 6, which forms the plug 8 capable of at least a radial outward expansion independently from the remaining part of the tampon 6. More generally, the tampon 6 has a proximal half and a distal half, and the covering covers said distal half and a portion but not all of said proximal half of said tampon, leaving an uncovered proximal part of said proximal half forming the proximal plug 8.

By virtue of this construction, the proximal plug 8 belonging to the tampon 6 is able to collect any liquid or fluid present in the intracorporeal cavity 2 and/or a first part of the active liquid running downward along the device 1, between the covering 7 and the mucous membrane 20 of the intracorporeal cavity. On account of this collection, the plug 8, made of porous material as described above, expands radially outward and thus blocks the entire device, at its lower or proximal part, in position in the intracorporeal cavity 2, while the substrate of the covering 7 and/or the wall 4 liquefies.

The withdrawal string 9 is attached to the proximal part or end of the device 1, e.g. to the capsule 3 and/or the tampon 6. As shown by Figure 1, the withdrawal string 9 is attached to the proximal portion 1a of the device, e.g. in a proximal portion of the tampon 6 or capsule 3. The withdrawal string 9 is a loop, with or without a knot, freely movable through the capsule 3 and/or the tampon 6. As shown by Figure 1, the proximal portion 6c of the tampon 6 comprises a through hole 24 designed for the free passage of at least one, if not two or more strands, 9a, 9b of the withdrawal string 9.

After insertion of the device 1 described with reference to Figure 1, and once positioned in the intracorporeal cavity 2 as shown in Figure 2, the different phases of functioning of a device according to the invention are shown in Figures 3 to 6 respectively.

The covering 7, if made of a low friction coating, makes it possible to limit or eliminate contact between the mucous membrane 20 of the body cavity 2 and the absorbent material when the tampon 6 is in the dry state at the time of insertion of the device 1; such contact could not only give rise to a not significant amount of friction but may also be a source of discomfort or pain to the user. On the other hand, by virtue of the configuration shown, almost the whole of the device, covered with the relatively slippery covering 7, slides in contact with the mucous membrane of the body cavity as the device is being inserted.

According to Figures 3-4, given the moisture (liquid or fluid) present in the intracorporeal cavity 2, a relatively small initial quantity of liquid phase (bodily liquid or fluid present in the cavity 2 and/or starting liquid form of the substrate of the covering 7 and/or the wall 4) flows from the distal end of the device 1, between the covering 7 and the mucous membrane 20 of the cavity 2, downward and along said covering 7. This start of liquid phase is collected and absorbed in the plug 8, which causes its radial expansion outward toward the mucous membrane 20 of the cavity 2. From the time of this expansion, the device 1 is held in position in the cavity 2, while at the same time sealing off the latter beyond the proximal end 1a of the device 1, with respect to the aforementioned liquid phase.

As shown in Figures 4-5, given the radial centripetal pressure opposed by the intracorporeal cavity 2, the tampon 6 is itself compressed. The distal part of the covering 7 and/or the distal part of the wall 4 of the capsule 3 liquefies, beginning to release the liquid charge 5 present in the capsule 3. The active liquid thus formed then flows from the capsule 3 onwards, ahead of the distal end 1b of the device 1, inside the intracorporeal cavity 2, and toward the proximal end 1a of the device 1, between the covering 7 and the mucous membrane 20 of the cavity 2. This flow is promoted by the expansion and compression of the tampon 6, and pressurization of the inside of the capsule 3, in particular through its tail 3b.

As shown in Figure 6, the active liquid phase arriving in the plug 8 allows the expansion of the latter to continue, said expansion having an effect within the tampon 6, pressurizing the liquid charge 5 in the capsule 3. The flow of the active liquid continues between the device 1 and the mucous membrane 20 of the intracorporeal cavity 2, as described above.

Finally, as shown in Figure 6, the covering 7 splits open completely, tearing if need be under the effect of the radial centrifugal pressure of the tampon 6. The latter fully expands radially, expelling practically all of the liquid charge 5 originally present in the capsule 4, the latter having virtually disappeared.

Thus, a device 1 according to the present invention brings with it an efficient method of transferring and/or circulating an active liquid into and/or inside an intracorporeal cavity 2, by the simple insertion of the device into and/or in contact with the mucous membrane 20 of the cavity 2, which automatically initiates the liquid phase to be released from the source 21 in the cavity 2.

As an example, a device 1, as previously described, can be left in said intracorporeal cavity 2 for less than one hour to several days, and preferably for several hours such as three to five hours, and then removed from said cavity.

The embodiments shown in Figures 7 to 10 differ from the embodiment with reference to Figure 1 in that the distal and peripheral portion 6d of the tampon 6 has, around its axial hole 6a, at least two longitudinal or axial slits 22, each passing through said peripheral part, and forming between them at least two parts or tongues 10 designed to expand, in particular axially or longitudinally, independently of one another, and possibly in a differentiated manner from one another.

According to Figures 7 and 8, there are two diametrically opposite longitudinal slits 22 forming between them two opposite tongues or parts 10. According to Figures 9 and 10, there are four cross slits 22 forming between them four tongues or parts 10. Other numbers of slits and tongues, e.g., 3, 5, 6 or more, could also be formed in the device.

As is shown more particularly in Figure 11, the arrangement previously described with reference to Figures 7 to 10 makes it possible to arrange a device according to the invention in the vaginal cavity 2, until it bears against the cervix 11. One or more tongues 10, specifically referred to 101, then remain in longitudinal or axial abutment against the cervix 11, while one or more other tongues, specifically referred to 102, can continue to expand longitudinally until they seal the cervix, as is shown in Figure 11.

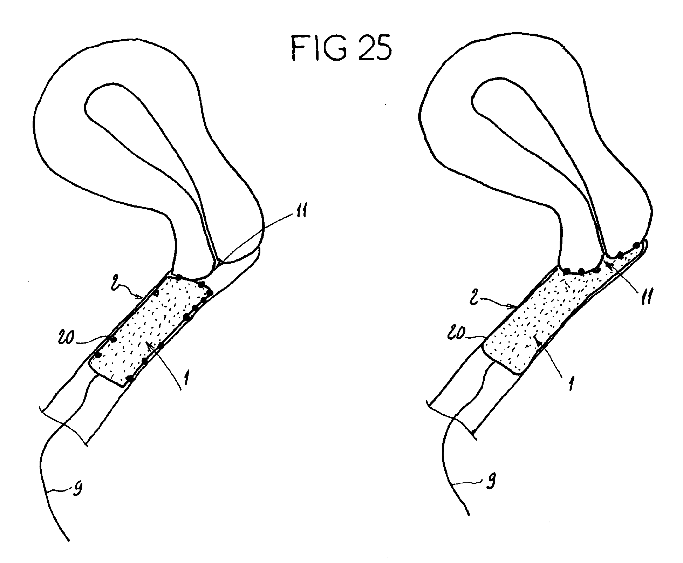

As shown by Figure 25, a device according to Figure 2 can be said normal, whereas a device according to Figure 11 can be said anatomical, in that in its expanded configuration it fits in addition with the anatomy of the cervix.

Thus, according to Figure 11, at least one tongue 101, 102 expands longitudinally or axially to seal and/or abut the cervix 11.

The number, shape, length and expandability of the tongues 10, 101, 102 shown by Figures 7 to 11 can be selected and controlled in accordance with aspects of the invention. For example, in certain embodiments of the invention, it is preferred that the cross-sectional area of the tongues 10 is less than that of the remainder of the body of the tampon 6. The location of the tongues 10 can be symmetrical, for example as shown Figures 7 to 10, or offset. The ends of the tongues 10 can be differently shaped, for example perpendicular to the expandable member axis, or at one or more angles to that axis or curved or the like.

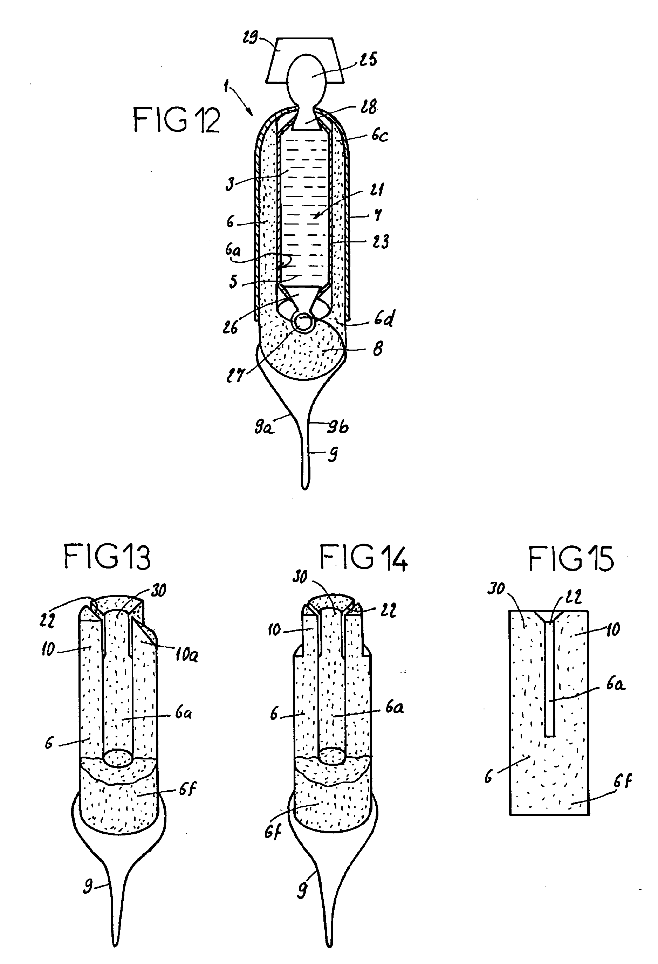

According to Figure 13, the tongues 10 are defined by shorter slits 20 than in Figure 9, and in which the top of tongue 10a is sloped rather than perpendicular with respect to the axis of the device. Preferred tongue lengths include but are not limited to about 0.5 to about 2.5, such as about 1 to about 2 cm.

A device according to the embodiments respectively shown by Figures 7-8, 9-10 and 11 can be made or manufactured in various ways. In particular, the expandability features of the tampon 6, including the tongues 10, 101 and 102 can be achieved in various ways.

In preferred embodiments of this invention, the body 6f of the expandable tampon 6 is first radially compressed, following which the area of tongues 10 is axially compressed. However, the opposite order or simultaneous compression are also possible. In an exemplary embodiment, a polygonal or circular cross-section body 6f of the material of expandable tampon 6 (see 6f in Figure 15) is compressed in a die or other compression member to achieve a desired diameter. Before, during or preferably after, such compression, a center core member 35 can be inserted into an end of the expandable tampon 6 to form the axial hole 6a. This can be achieved with or without previously forming a primary hole in the location of hole 6a. The body of the expandable tampon 6 is preferably compressed symmetrically, although symmetrical compression is not required. For example, it may first be compressed in one direction, followed by compression in another direction, or may be simultaneously or sequentially compressed from 2,3 4 or more or all directions.

In a preferred embodiment, as shown in Figure 14, the tongues 10 have a reduced cross-section relative to the body of expandable tampon 6. This can facilitate, for example, their extension into narrowed portions of the vaginal cavity 2 in the vicinity of the cervix 11 (refer to Figure 11). In an example of such an embodiment, the body 6f is radially inwardly compressed without inwardly radially compressing the tongue area 30. The reduced cross-sectional area of the tongue area 30 can be formed either prior to, simultaneously with or following compression of the body area 6f.

As an example of a preferred manufacturing process and apparatus, the following process and apparatus will be described. However, variations on this process and apparatus, as described below and as would be understood by one of ordinary skill in the art, are encompassed by the invention.

The following description will focus on manufacture of an expandable tampon 6 as shown in cross-section in Figure 15.

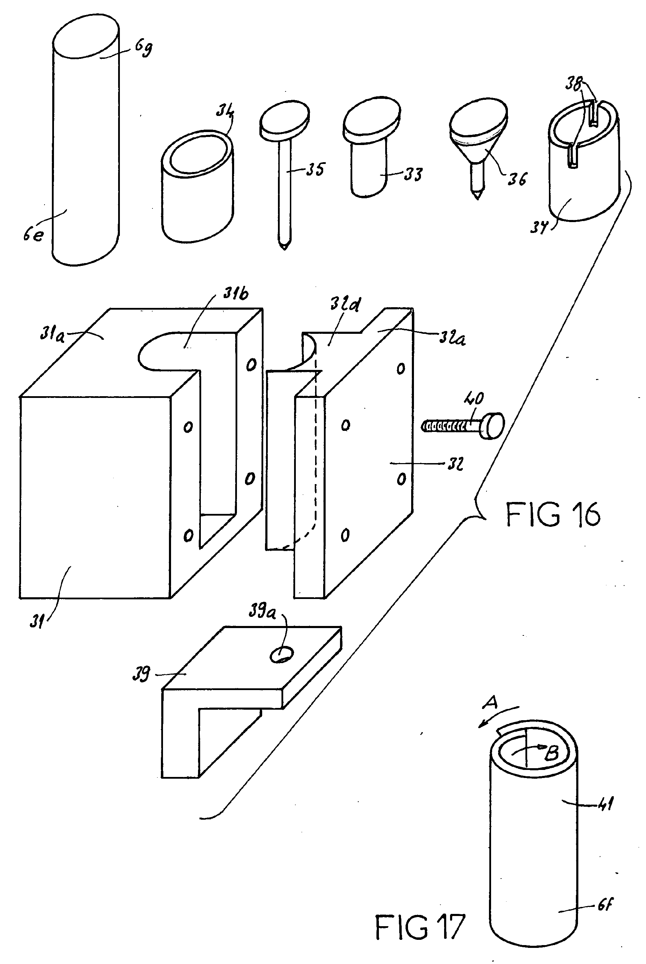

Referring to Figure 16, a preformed cylinder 6e of the absorbent material of the expandable tampon 6 may be inserted into slot 31 b of block 31. In this embodiment, the cylinder 6e must be compressed from two sides to fit into slot 20b. Preferably, an end 6g of cylinder 6e extends above top face 31 a of block 31.

Compression member 32 is then moved toward block 31 so that extension 32d slides into slot 31b to compress the cylinder 6e into a radially smaller cylindrical shape. Threaded members 40, for example, can be used to force bock 31 and compression member 32 together.

While cylinder 6e is compressed between block 31 and compression member 32, with end 6g remaining relatively uncompressed above faces 31 a and 32a, vertical compression member 33 may be lowered to compress end 6g longitudinally. If compression member 33 has the same diameter as the cylinder 6e within block 31 and compression member 32, forcing it downward to compress end 6g will also tend to result in cutting off an outer ring of end 6g, leaving the inner member of end 6g longitudinally compressed but substantially not radially compressed. Optionally, annular member 34 can be used to support end 6g while member 33 is lowered through member 34, thereby preventing tilting or lateral movement of end 6g during longitudinal compression. As an alternative to having member 33 cut end 6g, end 6g can be previously formed to have a smaller diameter than body 6f. The end of compression member 33 may be sloped or shaped as desired to form an end shape of the compressed body 6f.

Compression member 33 may then be moved away and core member 35 can then be driven into the end of the compressed cylinder 6e. This can form the axial hole 6a, while simultaneously providing radial compression from inside cylinder 6e. Alternatively, member 33 could be inserted into cylinder 6e before or while the same is compressed between block 31 and compression member 32.

Finally, conical member 36 can be forced downward into the compressed cylinder, preferably directed through the hole formed by core member 35, thereby forming the conical shape of Figure 15, at the distal end of the tampon, which accommodates the proximal bottom of the capsule 3 shown, for example, in Figure 1.

A guide member, for example guide member 39 of Fig. 16, can be attached to, e.g., block 31 to ensure controlled movement of the various members 33, 35 and 36, e.g., through hole 39a.

The compressed cylinder 6e can be removed from block 31 and compression member 32, for example, by backing compression member 32 away from block 31. Slits 22 can then be formed in the distal end of the compressed cylinder 6e, for example, using a guide 37 with slots 38 of predefined number and length and moving a blade down the slots. Slots can be of the same or different lengths, selected according to the goals for the device.

To complete formation of the device 1, a capsule 3 can be inserted into the hole 6a formed in the body 6, and then the capsule 3 and body 6 can be coated by the covering 7 as described herein.

The covering 7 can be applied by known coating techniques, preferably with the covering substrate being in a liquid form, such as molten or dissolved or dispersed in a suitable solvent. Dip-coating is an exemplary method of applying the covering. A preferred method for applying the covering constitutes spraying the substrate material onto the device comprising the tampon 6 and the source 21 of liquid. This can achieve good control of the covering thickness, and preferably form a very thin covering layer.

When exposed to moisture, the body 6 expands radially inwardly and outwardly, and the tongues 10 expand longitudinally, all regaining substantially the original dimensions to the extent permitted by the body cavity 2 into which the device 1 is inserted.

By following the manufacturing method previously described, and with reference to the specific embodiment shown by Figure 11, the expansion rate of each of the tongues or parts 101 and 102 in the radial (inward and/or outward) or transverse direction to the device 1, and/or in the axial or longitudinal direction of the device 1, can be controlled; for instance by adjusting or predetermining the volume, e.g. dimensions, and/or the compression rate in the dry slate of the absorbent material, in said radial direction, inwards and/or outwards, and/or in said axial direction.

For instance, with reference to Figure 11, the axially expandable part or tongue 102 may be less radially expandable than the other expandable part or tongue 101, in the distal portion 6d of the tampon. To this end, for instance, the part or tongue 102 would have a smaller cross-section when expanded than does the other part or tongue 101 of the distal portion 6d of the expanded tampon 6.

As noted above, variations in the apparatus and procedure are readily contemplated. For example, in addition to the variations described above, radial compression of the body 6e could be accomplished by a spiral compression device as shown in Figure 17, corresponding in general nature to an oil filter wrench. Thus, means, known but not shown, such as threaded members and matching holes, sliding parts, clamps or the like, are provided for moving the outer portion of the spiral in the direction of arrow A and/or the inner portion of the spiral in the direction of arrow B to compress a body 6 within the central opening of the cylinder 41. The compressed cylinder 6 can be treated as described above, again optionally with a core 35 being placed in the cylinder before or after it is compressed. The compressed cylinder 6 can be placed in a support and/or retained in cylinder 41, while being further processed. The above processes may be automated, for example using microprocessor controls.

In an aspect of the invention, the expandable tampon is coated with a biocompatible hydrophobic or liposoluble substance such as an oil before covering substrate is applied to it. This provides at least two significant benefits. First, it prevents the covering substrate from interacting with the material of the tampon to cause it to expand or apply pressure to the envelope prematurely. Second, it can provide a lubrication effect to any portion of the tampon 6 that is not covered by the covering, thus improving the feel of the device to the user.

The second embodiment shown in Figure 12 differs from the first embodiment only in the following respects.

The capsule 3 comprises a plastic envelope or film 23, for instance made of biocompatible plastic material, containing the liquid charge 5 of said liquid phase enclosed by said envelope in a leak-tight manner relative to the outside of said capsule, an active agent being distributed as previously in the liquid charge.

The capsule 3 is almost enclosed or contained in the tampon 6, leaving only its distal end accessible to the user. To this end, the blind and axial bore 6a of the tampon 6 is tailored in shape and dimensions to the filled envelope 23 of the capsule 3.

The envelope 23 of the capsule 3 is sealed at its proximal end by an internal plastic stopper 26, on which the envelope 23 is weld or sticks, and from which emerges outside a ring 27 contained in the bore 6a of the tampon at its bore and proximal end.

The envelope 23 of the capsule 3 is sealed at its distal end by an opening element 25 extending from the source 21 of liquid phase distal to the distal end of the tampon 6, comprising a separable appendage 28 formed at the distal end of the plastic envelop 23, partly outside of the tampon 6 with a handling wing 29, said separable appendage communicating with the inside of the envelope.

The withdrawal string 9 is attached directly to the envelope 23, or indirectly through the ring 27.

The working of the second embodiment shown in Figure 12 differs from the working of the first embodiment, only in the following respects.

Just before the device 1 is inserted into the intracorporeal cavity 2, the opening element 25 is removed by breaking it off, unscrewing it, or by any other manner or means. The capsule 3 is thus just open at its distal end before the device 1 is inserted into the intracorporeal cavity 2, and a small amount of the liquid charge 5 is immediately available to moisten the exterior of the device 1 at the time of inserting it into said cavity.

According to the present invention, it has also been discovered that reducing absorption of the active liquid dispensed in the intracorporeal cavity by at least a portion of the tampon 6 makes it possible to obtain various advantages which are set out herein.

The absorption capacity of the tampon 6 may be reduced by using one or more absorption barriers, consisting in closing, leveling and/or filling at least the surface pores and possibly the internal pores of the tampon 6, or consisting in filler materials.

Most preferably, the absorption barrier comprises or consists in a coating of a repelling substance, not miscible with the active liquid, coating the portion of the tampon 6 having a reduced absorption. This repelling substance is preferably hydrophobic where the active liquid is hydrophilic, or hydrophilic where the active liquid is hydrophobic.

The absorption barrier, for instance a coating of a repelling substance, acts in a differentiated manner resulting in at least one non-absorbing zone of the tampon 6 showing a reduced absorption capacity of the active liquid, and in at least one absorbing zone of same tampon 6, maintaining the absorption capacity of the active liquid.

According to the present invention, a differentiated impregnation of the tampon 6 with a repelling substance, e.g. an oily phase not miscible with the liquid phase of the active liquid, is hereunder described.

Differentiated impregnation is to be understood as meaning the fact that in a predetermined manner the repelling substance saturates the absorbent material in certain zones of the tampon 6, for example superficial zones of the latter, thereby rendering them nonabsorbent or less absorbent for the active liquid. The absorption capacity of the same tampon 6 is maintained intact or equal in other zones, or remaining zones, with respect to the active liquid.

Preferably, in a manner not shown, the repelling substance, e.g. oily phase, impregnates a distal part of the tampon 6, with the exclusion of a proximal part of said tampon, comprising the proximal plug 8.

This type of impregnation affords three advantages:
- it allows the active liquid to be in contact with the mucous membrane 20 of the intracorporeal cavity 2 for a longer time, since in particular said liquid is practically unabsorbed at the site of its release, that is to say at the distal end of the device 1,
- by saturating a part of the tampon 6, it is possible to limit the absorbed quantity of the active liquid, and consequently the increase in the volume of the liquid charge 5 in the capsule 3,
- the phenomenon of absorption is thus directed on the proximal part of the tampon 6.

However, according to a variant of this invention, the coating of the repelling substance can extend over a restricted part of the proximal plug 8.

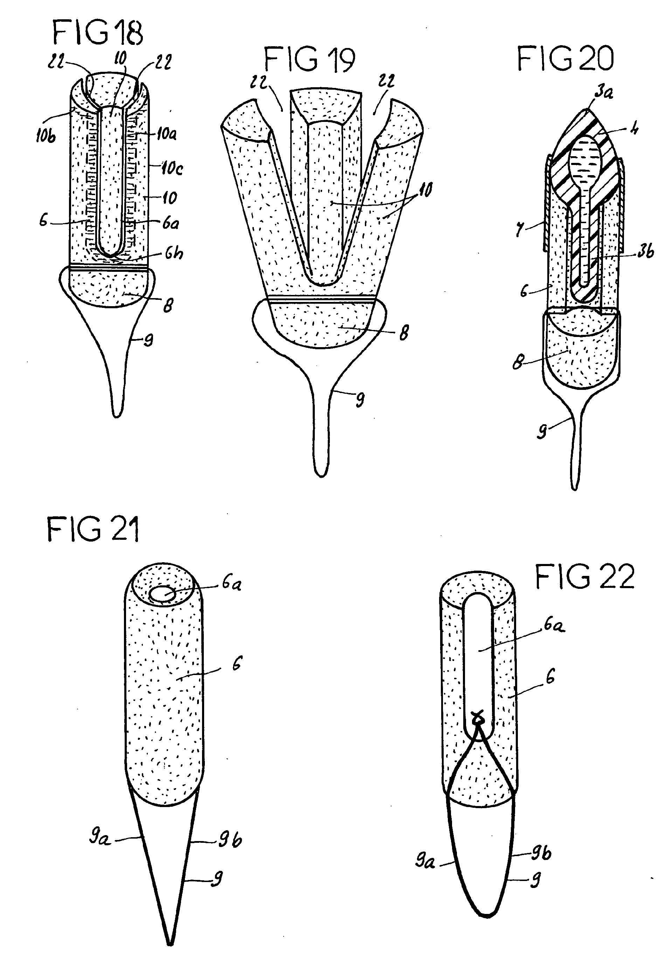

Preferably, but in a nonlimiting manner, and as is shown in Figure 18, the repelling substance impregnates to a limited depth the inner 10a and frontal 10b faces of each tongue 10, as well as the rear face 6h of the bottom of the blind axial hole 6a, the rear face 10c and possibly the core of each tongue.

By virtue of this arrangement, and as is shown in Figure 19, once the tampon 6 is impregnated by the active liquid, the branches 10 open out in a corolla in a directed and controlled manner, and without acting aggressively on the mucous membrane 20 of the intracorporeal cavity 2.

In a manner not shown, the repelling substance can impregnate some of the tongues 10, to the exclusion of the others, in such a way as to obtain a preferred and directed expansion of said tongues relative to the other ones.

By way of example, the oily phase is lanolin, as a repelling substance.

With reference to Figure 11, differentiating the tongues 101, 102 according to their capacity to absorb the active liquid by differentiated impregnation with the repelling substance makes it possible to direct the expansion of the distal part of the tampon 6 in a manner adapted to the neck of the uterus, in particular to reach the latter in all relative positions of the device 1 according to the invention, with respect to the intracorporeal cavity 2.

The repelling substance can also impregnate portions excluding the tongues 10.

By way of example, the string 9 for extracting the device 1 is inserted in a loop shape in the tampon 6 of absorbent material, as has previously been described, and repelling substance also impregnates said string.

As is shown in Figure 20 the covering 7 for temporary protection may envelop only the proximal part of the capsule 3 and the distal part of the tampon 6.

In this way, the capsule 3 can open out very quickly, allowing the active liquid to remain as long as possible in contact with the mucous membrane 20 of the intracorporeal cavity 2, in particular the neck of the uterus.

This arrangement also makes it possible to reduce the volume of the substrate, present in the device according to the invention, while at the same time permitting or favoring the corolla-like opening of the tampon 6 at the distal end of the device 1, as has been previously described.

As is shown in Figure 20, the distal end of the capsule 3 remains free in relation to the covering 7 for temporary protection.

The principles of the invention described above can also be implemented using a device such as that shown in Figure 12.

In the embodiment of Figures 7 to 11, the repelling substance, e.g. an oily phase, can be impregnated into the distal section of the tongues 10, extending along the reference axis 15 from the frontal distal face 10b to an intermediate level comprised between said distal face 10b and a bottom 6h of said tongues. Preferably, at least a tip of the tongues is impregnated, for instance one or two millimeters in depth, and in embodiments over about a fourth or a third to a half of their length. This can readily be accomplished, for example, by dipping the distal section of the tampon into oil, or spraying oil onto the distal end of the tampon or the like. For example, the distal section of the tongues may be impregnated in their compressed configuration with at most about a centimeter of oil such as lanolin oil for this purpose.

A delivery device according to this last example has been tested with an animal model, with
six prototypes, i.e.:
- three samples comprising a capsule containing a water solution mixed with toluidin blue and methylen blue,
- one sample comprising a capsule containing glycerin with methylen blue,
- two samples comprising a capsule containing an isopropyl myristate solution mixed with soudan black stain were introduced into a vaginal cavity in adult sheep (and in one case of a pig),.

After three to four hours, the animals were sacrificed for external and internal vaginal examination.

The results pointed out that:
- complete deployment of the tongues was obtained,
- no posterior migration of the prototypes were observed; all of them were in contact with the cervix,
- no mucosal staining was observed behind the proximal part of the prototype, whereas the area of the vaginal mucosa located ahead of the distal end of the same prototype was uniformly stained in blue or black; just a few and well limited folds were poorly stained in 2/6 cases with the water solution,
- a specific prototype allowed, in the pig model, the isopropyl myristate solution to penetrate into the linear cervix (swine) over a distance of 8 cm over the distal end of the prototype,
- the cervix itself was entirely and uniformly stained with toluidin/methylen blue or soudan black.

It has thus been found that such an arrangement helps maintain the liquid charge, particularly a liquid charge comprising isopropyl myristate, in the desired area of the body cavity 1 distal to the body of the tampon and avoids absorption of excess liquid charge by the tampon 6, maximizing its availability to the body tissues, both in terms of volume and of time.

In the arrangement of Figures 21 and 22, the string 9 enters and exits the tampon 6 through the proximal end of the tampon rather than through the sides of the tampon. As a result, the smoothness of the sides of the tampon 6 is maintained and any discomfort from the thread rubbing against the mucous membrane 20 during insertion and removal is avoided, and the resistance of the tampon to cutting by the string is improved. In addition, the knot in the looped string 9 is preferably arranged to be inside the tampon. This improves the aesthetic appearance of the device, and avoids any discomfort arising from movement or pressure of the knot against the cavity walls.

In addition, the proximal face of the tampon 6 may be treated to limit or prevent absorption of fluids or selected fluids in a similar manner as discussed above. In such embodiments, it is preferred that only the proximal surface, and little or none of the sides or depth of the tampon be rendered less or non-absorptive. Such treatment may be desirable to act as a physical barrier against contamination, for example during swimming or sports activities, and is preferably accomplished by a surface treatment, without impairing the expandability of the proximal portion of the tampon comprising the plug.

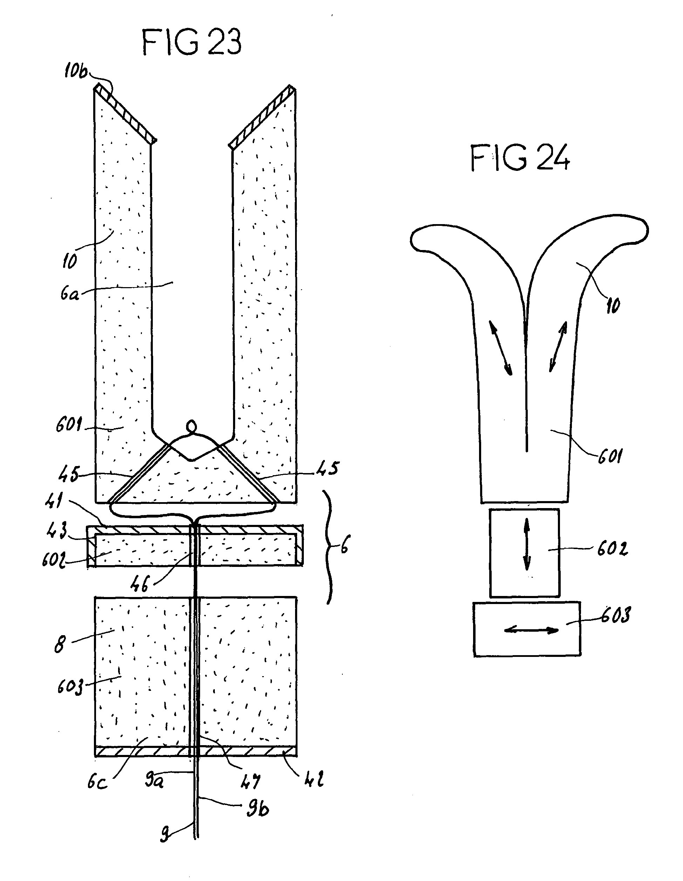

An embodiment represented by Figures 23 and 24 differs from the embodiments previously described, mainly in that the tampon 6 is a composite part or component, obtained by assembling three distinct sections, i.e.:
- a distal section 601 comprising the tongues 10, which in expanded state exhibit the corolla shape shown in Figure 24, and which is made of cellulose viscose sponge,
- an intermediate section 602 exerting an axial thrust in expanded state, as shown by Figure 24, which is made of a plastic foam having a shape memory,
- and a proximal section 603 comprising the plug 8 previously described, exerting a radial and outward thrust in expanded state, as shown by Fig. 24, which is made of a cellulose viscose sponge.

The intermediate section 602, between the distal 601 and proximal section 603 is enclosed in a ring 43 of a liquefiable substrate, as previously defined, for instance an alginate.

The sections 601 and 603 are made of an absorbent material, as previously defined, and manufactured in a similar way to the way described with reference to Figure 16.

The composite structure of the tampon 6 shown by figure 23 is interrupted by transverse capillary flow isolating sections 41 and 42. Each of these sections may be an absorption barrier with respect to the active liquid as previously defined, in particular a repelling substance, e.g. an oily liquid, also as previously defined. Each of these isolating sections lies over the whole cross-sectional area of tampon 6, and is actually a barrier or screen toward any capillary flow within the tampon 6 in the axial direction.

One transverse isolating section 41 is intermediate between the proximal plug 8 and the distal portion of the tampon. More precisely, the isolating section is located on the distal face of the intermediate section 602, against the proximal face of the distal section.

Another isolating section 42 is located at the proximal end of the tampon 6, and more precisely on the proximal face of the proximal section 603.

As previously described with reference to Figure 18, the frontal distal face 10b of all the tongues 10 is covered by a capillary flow isolating or absorption barrier material, for instance a repelling substance, e.g. an oily phase.

The path for the free passage of the withdrawal string 9 is obtained by drilling:
- two opposite and oblique through holes 45 in the proximal part of the distal portion 601, each from the inside of the proximal face of the distal portion 601 to the axial hole 6a, these two holes 45 accommodating respectively two strands 9a and 9b of the string 9, which may or may not slide freely respectively in said holes,
- an axial through hole 46 in the intermediate portion 602, having a large section,
- an axial through hole 47 in the proximal portion 603, having a small section.

The two strands 9a and 9b then pass through the aligned holes 46 and 47, and emerge from the tampon 6 through its proximal end 7 or face 6c.

All of the components of device as shown by Figures 23 and 24, including the capsule 3 (not shown) can be assembled together by wrapping them in their assembled configuration with the covering substrate as previously described. If necessary, the assembling can be previously made firm or solid, with additional means such as previously gluing its components together.

Various modifications can be made in the devices and methods of the invention, for example combination of various ones of the described features. The specific embodiments described herein thus are intended to be illustrative and not exclusive.

More generally, the present invention discloses a method of transferring and/or circulating an active liquid into and/or inside an intracorporeal cavity (2), comprising inserting a device (1) into and/or in contact with the mucous membrane (20) of said cavity and allowing a liquid phase to be released from said source (21) in said cavity.

Preferably, said intracorporeal cavity (2) is a vagina.

Said liquid phase comprises an active agent comprising at least one member selected from the group consisting of wetting agents, solubilizing and liquefying agents of a bodily liquid or fluid present in said cavity, therapeutic agents against infectious and non infectious diseases, prophylactic agents, diagnostic agents, cosmetic agents, personal hygiene agents, antiseptic agents, bactericidal agents, fungicidal agents, spermicidal agents, local treatment agents, systemic treatment agents, trophic agents and lubricant agents.

As an example, the active agent is an immunotherapeutic agent.

As an example, the present invention discloses a method of transferring and/or circulating an active liquid into and/or inside an intracorporeal cavity (2), comprising inserting a device (1) as shown by Figures 9 and 10 into and/or in contact with the mucous membrane (20) of said cavity, and allowing a liquid phase to be released from the source (21) in said cavity, wherein at least one said tongue (101) expands longitudinally or axially to seal the cervix (11), and/or wherein at least one said tongue (102) expands longitudinally or axially to axially abut the cervix (11).

As an example, the present invention discloses a method of transferring and/or circulating an active liquid into and/or inside an intracorporeal cavity (2), comprising inserting a device (1) as shown by Figure 1 into and/or in contact with the mucous membrane (20) of said cavity and allowing a liquid phase to be released from the source (21) in said cavity, wherein said proximal plug (8) expands radially inside said cavity (2) while said substrate is at least partially liquefying.

Expansion of said proximal plug seals off movement of liquid beyond the proximal end (1a) of said device (1), holds the device (1) in position in the cavity (2), and pressurizes the inside of said capsule (3).

As an example, the present invention discloses a method of transferring and/or circulating an active liquid into and/or inside an intracorporeal cavity (2), comprising inserting a device (1) as shown by Figure 1 into and/or in contact with the mucous membrane (20) of said cavity and allowing a liquid phase to be released from the source (21) in said cavity.

As an example, the present invention discloses a method of transferring and/or circulating an active liquid into and/or inside an intracorporeal cavity (2), comprising inserting a device (1) as shown by Figure 12 into and/or in contact with the mucous membrane (20) of said cavity and allowing a liquid phase to be released from the source (21) in the cavity.

The liquid phase is expelled from said source (21) by expansion of said tampon (6).

As an example, the present invention discloses a method of transferring and/or circulating an active liquid into and/or inside an intracorporeal cavity (2), comprising inserting a device (1) as shown by Figure 12 into and/or in contact with the mucous membrane (20) of said cavity and allowing a liquid phase to be released from said source (21) in said cavity, further comprising removing the opening element (25) just before the device (1) is inserted into the intracorporeal cavity (2).

Whatever the example concerned, the device (1) is left in said intracorporeal cavity (2) for three to five hours, and then removed.

The present invention discloses also a method of making a device (1) comprising:
radially compressing (31, 32) at least a first portion (6e) of an expandable tampon (6) ;
axially compressing (33, 34) at least a second portion (6g) of said expandable tampon (6); and
   providing said expandable tampon with a source (21) of liquid phase.

Radially compressing said first portion (6e) of said expandable tampon (6) possibly comprises radially inwardly (31, 32) and radially outwardly (31, 35) compressing said first portion.

Possibly, said second portion (6g) of said expandable tampon (6) is substantially not radially inwardly compressed (33, 34).

Possibly, said first portion (6e) of said expandable tampon (6) is radially outwardly compressed (31, 32) after being radially inwardly compressed (33, 34).

Possibly, said first portion (6e) of said expandable tampon (6) is simultaneously radially inwardly and outwardly compressed (31, 32, 35).

Possibly, said second portion (6g) of said expandable tampon (6) is axially compressed (33, 34) after said first portion is radially inwardly compressed (31, 32).

Possibly, said second portion (6g) of said expandable tampon (6) is axially compressed (33, 34) after said first portion is inwardly radially compressed (31, 32) and before said first portion (6e) is radially outwardly compressed (31, 35).

Possibly, said first (6e) and second portions (6g) of said expandable tampon (6) are radially outwardly compressed (31, 35) after said second portion (6g) is radially inwardly compressed.

Possibly, said first (6e) and second portions (6g) of said expandable tampon (6) are radially outwardly compressed by piercing them with a core member (39).

Possibly, said second portion (6g) of said expandable tampon (6) is formed with a reduced cross-sectional area in an expanded condition relative to said first portion (6e).

Possibly, said reduced cross-sectional area is formed during said axial compression (33, 34).

Possibly, wherein said second portion (6g) has the same cross-sectional area as said first portion (6e) in a fully compressed condition.

Possibly, the making method further comprises covering at least a portion of said device (1) comprising said expandable tampon (6) and said source (21) of liquid with a covering liquefiable substance that is relatively solid in ambient temperature and atmosphere, but that can at least partially enter a liquid state when in intracorporeal said cavity (2).

The present invention discloses also an apparatus for making a device (1) comprising:
means (31, 32) for radially compressing at least a first portion (6e) of an expandable tampon (6);
means (31, 33) for axially compressing at least a second portion (6g) of said expandable tampon; and
means for providing said expandable tampon (6) with a source (21) of liquid.

Possibly, said apparatus further comprises means for covering at least a portion of said expandable tampon (6) with a liquefiable substance that is relatively solid in ambient temperature and atmosphere, but that can at least partially enter a liquid state when in an intracorporeal cavity (2).

As an example, said apparatus comprises:
a die (31, 32) configured to radially compress at least a first portion of an expandable tampon (6);
a die (32, 33) configured to axially compress at least a second portion of said expandable tampon (6); and
a loader configured to provide said expandable tampon with a source (21) of liquid.

As an example, said apparatus comprises further comprises a sprayer configured to cover at least a portion of said expandable tampon (6) with a liquefiable substance that is relatively solid in ambient temperature and atmosphere, but that can at least partially enter a liquid state when in an intracorporeal cavity (2).

As an example, said apparatus further comprises a core member (35) configured to radially outwardly compress at least a third portion of said expandable tampon (6).

As an example, said third portion of said expandable tampon (6) comprises said first (6e) and second portions (6g).

## Claims

1. A disposable device for transferring and/or circulating an active liquid into and/or inside an intracorporeal cavity, said device being elongate along a reference axis, said device having an intrinsic axial consistency which is sufficient to allow said device to be introduced into said cavity by being pushed in axially, said device comprising:
an expandable tampon made of at least one absorbent material, having a proximal portion and a distal portion, designed to collect and absorb a liquid in the intracorporeal cavity and to expand and bear in a leak tight manner against a mucous membrane of the intracorporeal cavity; and
a source of liquid phase comprising a capsule, wherein at least a proximal part of the capsule is at least partially surrounded by said expandable tampon;
wherein at least one part of said distal portion of the tampon comprises at least two tongues that are independently axially expandable upon absorption of a liquid.

2. The device according to claim 1, wherein at least one said tongue is configured to expand axially to axially abut a cervix.

3. The device according to claim 1, wherein at least one said tongue is configured to expand axially to seal a cervix.

4. The device according to claim 3, further comprising:
at least one absorption barrier, said absorption barrier comprising at least one member selected from the group consisting of closed, leveled, and filled surface pores of the tampon, and
wherein the liquid phase comprises an active liquid, said active liquid comprising an active agent comprising at least one member selected from the group consisting of wetting agents, solubilizing and liquefying agents of a bodily liquid or fluid present in said vagina, therapeutic agents against infectious and non infectious diseases, prophylactic agents, diagnostic agents, cosmetic agents, personal hygiene agents, antiseptic agents, bactericidal agents, fungicidal agents, spermicidal agents, local treatment agents, systemic treatment agents, trophic agents and lubricant agents,
wherein said absorption barrier reduces absorption of said active liquid by at least a portion of the tampon.

5. The device according to claim 4, wherein said absorption barrier comprises a coating of a repelling substance coating at least said portion of the tampon.

6. The device according to claim 5, wherein the repelling substance is impregnated in at least one part to a limited depth of at least one said tongue, said at least one part being selected from the group consisting of an inner face, a frontal distal face, a rear face, and a core of said at least one tongue.

7. The device according to claim 5, wherein the repelling substance is impregnated in at least a distal section of at least one said tongue extending along said reference axis from a frontal distal face of said tongue to an intermediate level between said distal face and a bottom of said tongue.

8. The device according to claim 5, wherein the repelling substance is impregnated in all of the tongues.

9. The device according to claim 5, wherein the tampon is configured with said tongues differentially impregnated with a repelling substance, wherein at least one said tongue differentially expands relative to at least one other said tongue.

10. The device according to claim 6, wherein at least one said tongue impregnated with the repelling substance is configured to keep said active agent at a cervix.

11. The device according to claim 5, wherein the repelling substance is impermeable to the active agent.

12. The device according to claim 1, wherein at least one said tongue is configured to expand axially to deliver an active liquid to a cervix.

13. The device according to claim 1, wherein at least one said tongue is configured to maintain an active liquid distal in relation to the tampon while the at least one said tongue expands axially.

14. The device according to claim 1, wherein said capsule comprises:
a wall made of a liquefiable substrate that is relatively solid at ambient temperature outside of the intracorporeal cavity and that becomes relatively liquid inside said intracorporeal cavity, so that said substrate at least partially liquefies inside said intracorporeal cavity and said wall disrupts;
a liquid charge, enclosed by said wall in a leak tight manner relative to the outside of said capsule; and
an active agent distributed in at least one of said substrate and said liquid charge.

15. The device according to claim 1, further comprising a covering for temporary protection, the covering at least partially covering a distal portion of said capsule, and extending longitudinally to an intermediate level of the tampon in such a way as to form in said tampon a proximal plug not covered by said covering, wherein the proximal plug is at least radially expandable independently from the remaining part of said tampon.

16. The device according to claim 15, wherein the covering is made of a substrate which is relatively solid at ambient temperature, outside the intracorporeal cavity, and which becomes relatively liquid inside said intracorporeal cavity, so that said substrate at least partially liquefies inside said intracorporeal cavity.

17. The device according to claim 16, wherein the liquefiable substrate of said covering at least partially melts and/or dissolves under conditions in said intracorporeal cavity.

18. The device according to claim 16, wherein the substrate of said covering comprises an alginate.

19. The device according to claim 4, wherein the tampon is configured with one or more said absorption barriers in a predetermined and differentiated manner, wherein the tampon has at least one non-absorbing zone having a reduced absorption capacity for the active liquid, and at least one absorbing zone having an absorption capacity at least approximately equal to the absorption capacity of the absorbent material for the active liquid.

20. The device according to claim 5, wherein said repelling substance is hydrophobic.

21. The device according to claim 5, wherein said coating extends over a part of said proximal plug.

22. The device according to claim 5, wherein the repelling substance is impregnated in a distal part of the tampon, with the exclusion of a proximal part of said tampon comprising the proximal plug.

23. The device according to claim 5, wherein said repelling substance is non-miscible with said active liquid.

24. The device according to claim 1, further comprising at least one transverse capillary flow isolating section comprising an absorption barrier extending transversally to the tampon.

25. An expandable tampon, comprising:
a proximal portion and a distal portion, the tampon being elongate along a reference axis;
wherein at least one part of said distal portion of the tampon is differentially axially expandable upon absorption of liquid.

26. The expandable tampon according to claim 25, wherein said at least one part of said distal portion of the tampon comprises at least two tongues that are independently axially expandable.

27. The expandable tampon according to claim 26, wherein said distal portion of said tampon has a periphery comprising at least two longitudinal slits passing through said portion and forming between them said at least two tongues.

28. The expandable tampon according to claim 27, further comprising a repelling substance impregnated in at least one said tongue to a limited depth, in at least one portion selected from the group consisting of an inner face, a frontal distal face, a rear face and a core of said at least one tongue.

29. The expandable tampon according to claim 28, wherein the repelling substance is hydrophobic.

30. The expandable tampon according to claim 27, further comprising a repelling substance impregnated in at least a distal section of at least one said tongue extending along said reference axis from a frontal distal face of said tongue to an intermediate level between said distal face and a bottom of said one tongue.

31. The expandable tampon according to claim 30, wherein the repelling substance is hydrophobic.

32. The expandable tampon according to claim 27, further comprising a repelling substance impregnated in all of the tongues.

33. The expandable tampon according to claim 32, wherein the repelling substance is hydrophobic.

34. The expandable tampon according to claim 27, wherein said tampon comprises:
a distal section comprising said tongues;
a proximal section comprising a proximal plug, wherein the proximal plug is at least radially expandable independently from the remaining part of said tampon; and
an intermediate section between said distal and proximal sections, enclosed in a ring.

35. The expandable tampon according to claim 25, wherein said distal portion of said tampon is subdivided into at least four parts that are independently axially expandable.

36. The expandable tampon according to claim 25, further comprising a withdrawal string attached in said proximal portion of said device.

37. The expandable tampon according to claim 36, wherein said withdrawal string is a loop freely movable through said tampon.

38. The expandable tampon according to claim 37, wherein the proximal portion of said tampon comprises an axial hole designed for the free passage of two parallel strands of said withdrawal string.

39. The expandable tampon according to claim 25, wherein said proximal portion is not axially expandable upon absorption of a liquid.

40. A tampon having a proximal portion and a distal portion, the tampon being elongate along a reference axis, and having an absorption barrier that reduces passage of liquid through at least a portion of the tampon.

41. The tampon of claim 40, wherein said absorption barrier comprises a layer of a repelling substance transverse to said reference axis.

42. The tampon of claim 41, wherein said repelling substance is hydrophobic.

43. A disposable device for transferring and/or circulating an active liquid into and/or inside an intracorporeal cavity, said device being elongate along a reference axis, said device having an intrinsic axial consistency which is sufficient to allow said device to be introduced into said cavity by being pushed in axially, said device comprising:
an expandable tampon made of at least one absorbent material, having a proximal portion and a distal portion, designed to collect and absorb a liquid in the intracorporeal cavity and to expand and bear in a leak tight manner against a mucous membrane of the intracorporeal cavity;
a source of liquid phase comprising a capsule, wherein at least a proximal part of the capsule is at least partially surrounded by said expandable tampon; and
a covering for temporary protection, the covering at least partially covering a distal portion of said capsule, and extending longitudinally to an intermediate level of the tampon in such a way as to form in said tampon a proximal plug not covered by said covering;
wherein said tampon comprises at least one transverse capillary flow isolating section.

44. The device of claim 43, wherein said transverse isolating section is intermediate between the proximal plug and a distal portion of said tampon.

45. The device of claim 43, wherein said transverse isolating section is located at a proximal end of said tampon.

46. The device of claim 43, wherein said transverse isolating section comprises an absorption barrier extending transversally to the tampon.

47. A method of making a disposable device for transferring and/or circulating an active liquid into and/or inside an intracorporeal cavity, said device being elongate along a reference axis, said device having an intrinsic axial consistency which is sufficient to allow said device to be introduced into said cavity by being pushed in axially, the method comprising:
radially compressing at least a first portion of an expandable tampon; and
axially compressing at least a second portion of said expandable tampon.

48. The method of claim 47, further comprising providing said expandable tampon with a source of liquid phase.

49. The method of claim 47, further comprising forming in said second portion at least two tongues that are independently axially expandable upon absorption of a liquid.

50. The method of claim 47, further comprising applying to said tampon a covering for temporary protection, the covering at least partially covering a distal portion of said capsule, and extending longitudinally to an intermediate level of the tampon in such a way as to form in said tampon a proximal plug not covered by said covering.

51. The method according to claim 47, wherein radially compressing said first portion of said expandable tampon comprises radially inwardly and radially outwardly compressing said first portion.

52. The method according to claim 47, wherein said second portion of said expandable tampon is less radially inwardly compressed than said first portion of said expandable tampon.

53. The method according to claim 51, wherein said first portion of said expandable tampon is radially outwardly compressed after being radially inwardly compressed.

54. The method according to claim 51, wherein said first portion of said expandable tampon is simultaneously radially inwardly and outwardly compressed.

55. The method according to claim 47, wherein said second portion of said expandable tampon is axially compressed after said first portion is radially inwardly compressed.

56. The method according to claim 47, wherein said second portion of said expandable tampon is axially compressed after said first portion is inwardly radially compressed and before said first portion is radially outwardly compressed.

57. The method according to claim 55, wherein said first and second portions of said expandable tampon are radially outwardly compressed after said second portion is radially inwardly compressed.

58. The method according to claim 57, wherein said first and second portions of said expandable tampon are radially outwardly compressed by piercing them with a core member.

59. The method according to claim 47, wherein said second portion of said expandable tampon is formed with a reduced cross-sectional area in an expanded condition relative to said first portion.

60. The method according to claim 59, wherein said reduced cross-sectional area is formed during said axial compression.

61. The method according to claim 59, wherein said second portion has the same cross-sectional area as said first portion in a fully compressed condition.

62. The method according to claim 47, further comprising covering at least a portion of said device comprising said expandable tampon and said source of liquid with a covering liquefiable substance that is relatively solid in ambient temperature and atmosphere, but that can at least partially enter a liquid state when in an intracorporeal cavity.

63. The method according to claim 47, further comprising forming slits in said second portion of the tampon after compression of the tampon.
